# EUROPEAN PATENT SPECIFICATION

(11) **EP 4 242 458 B1**
(45) Date of publication and mention of the grant of the patent: **10.07.2024**
(21) Application number: 21823626.3
(22) Date of filing: 09.11.2021
(51) Int. Cl.: F04B 43/12

(54) **PERISTALTIC PUMP FOR HYSTEROSCOPY**
PERISTALTIKPUMPE FÜR DIE HYSTEROSKOPIE
POMPE PÉRISTALTIQUE POUR HYSTÉROSCOPIE

(30) Priority: 09.11.2020 ES 202031125
(43) Date of publication of application: 13.09.2023
(73) Proprietor: Alonso Pacheco, Luis, 29630 Málaga (ES); Haimovich Segal, Sergio Mario, 29630 Málaga (ES); Carugno Matute, José Antonio, 29630 Málaga (ES); Rosado Rios, Juan, 29630 Málaga (ES)
(72) Inventor: Alonso Pacheco, Luis, 29630 Málaga (ES); Haimovich Segal, Sergio Mario, 29630 Málaga (ES); Carugno Matute, José Antonio, 29630 Málaga (ES); Rosado Rios, Juan, 29630 Málaga (ES)
(74) Representative: Isern Patentes y Marcas S.L.
(86) International application number: PCT/ES2021/070806
(87) International publication number: WO 2022/096769

(56) References cited:
- WO-A1-2009/073212
- CN-B- 105 508 210
- GB-A- 1 344 825
- GB-A- 2 069 063
- US-A1- 2009 087 326

## Description

### OBJECT OF THE INVENTION

The object of the present application is to provide a peristaltic pump for medical tests, such as for example hysteroscopy.

More specifically, the invention proposes the development of a fluid management system provided with a peristaltic pump for hysteroscopy which makes it possible to use lower cost disposable standard infusion tubes, made of polyethylene (PE), polypropylene (PP), PVC, etc. for its operation.

### BACKGROUND OF THE INVENTION

Hysteroscopy is a medical test in the field of gynaecology which consists of introducing a small camera into the uterine cavity. This test is used to diagnose intrauterine pathologies in patients, as well as to perform certain interventions inside the uterus.

Since the uterine cavity is a virtual cavity, it is necessary to instil a liquid into the uterus to separate the walls and thus visualise the uterine cavity. This instillation of fluids into the cavity is carried out by different means, fluid management systems being the most widely used. These systems enable the flow rate as well as the pressure of the liquid that is being introduced into the uterine cavity to be controlled at all times.

There are currently multiple models on the market for hysteroscopy and urology interventions. All models use a peristaltic pump to control the flow necessary to carry out the intervention and to also be able to control and limit the fluid pressure at all times. An example of a peristaltic pump is described in WO 2006016122.

These peristaltic pumps comprise a series of pressure rollers mounted on a rotary structure, arranged to press a flexible silicone tube, so that when each pressure roller rotates, it presses the tube causing a flow of fluid inside it. The flexible silicone tube occupies only the section of the pressure rollers of the pump, such that a fluid inlet tube and a second outlet tube towards the patient must be connected to this tube, which are disposable.

To control the pressure, sensors are used which are in contact with the fluid and the flow rate with the rotational speed of the rollers. However, in practice it is observed that the fact that these sensors are in contact with the fluid has a series of drawbacks. On the one hand, the sensors are integrated into the catheter system, wherein each peristaltic pump on the market uses its own catheter system, such that said specific catheter system must be purchased, which implies an increase in costs for using devices of this type.

On the other hand, these systems are single-use systems and therefore disposable. In other words, at the end of each intervention, the tube system must be replaced and exchanged for a new one, which also generates an added cost to each intervention.

Therefore, all this entails a considerable additional operating expense, since usually a single device performs a multitude of operations per day, sterilising or changing the aforementioned silicone tubes being necessary for hygienic reasons, the latter being a consumable with a high cost.

Documents no. GB 2069063, US 2012082576 and US 2009087326 are known which describe pumps that are equipped with release means for the fluid tube, the common features of which form part of the preamble of claim 1.

Furthermore, the applicant is currently unaware of the existence of an invention having all the features described herein.

### DESCRIPTION OF THE INVENTION

The present invention has been developed for the purpose of providing a peristaltic pump configured as a novelty in the field of the application and resolves the aforementioned drawbacks, also providing other additional advantages that will be evident from the accompanying description below.

An object of the present invention is therefore to provide a peristaltic pump of the type comprising a casing that houses drive means for acting on a rotor provided with a head that supports a plurality of pressure rollers distributed radially around the rotor, said pressure rollers being intended to make contact with pressure with a flexible tube for the passage of a fluid, the casing having a housing configured for accommodating the flexible tube. In particular, the invention is characterised in that the casing includes release means for releasing the flexible tube, having an arrangement such that in a closed condition of the release means, such release means are configured to keep the flexible tube in the housing in an immovable manner and the pressure rollers in contact under pressure with the flexible tube.

Thanks to these features, using a disposable silicone tube for driving the fluid is not required, such that it allows the use of any generic system for administering serum, which enables costs to be reduced. This is due to the fact that the flexible tube used for the passage of a fluid is independent of the peristaltic pump.

Preferably, the release means comprise a locking element articulated at an articulation point present in the casing, such that it can be manually rotated by a user with ease, having a configuration that runs along the housing for the flexible tube.

Advantageously, the locking element has a curved region configured for adapting to the contour of the head of the rotor that has a smooth inner surface.

Preferably, the inner portion of the locking element comprises a plurality of recessed regions configured for partially housing the flexible tube.

Additionally, the locking element includes clamping means configured for clamping the flexible tube, the curved region being devoid of the clamping means.

Preferably, the clamping means may comprise at least one section provided on the inner face thereof with a toothed region configured for coming into contact with the flexible tube when it is mounted in the housing present in the casing.

According to another aspect of the pump of the invention, pressure detection means are provided configured for detecting the pressure value of the fluid to run along the flexible tube, the detection means being located in the casing. The fact that the control system is external to the flexible tube, i.e., the control system is not in direct contact with the fluid to circulate through the inside of the flexible tube, enables maintaining the sterilisation, for example, of the fluid that is to come into contact with the uterine cavity.

In an exemplary embodiment, the detection means consist of a pressure sensor located at a point along the housing for the flexible tube, the locking element having a projection that protrudes from the inside thereof, being located such that in a closed condition of the locking element, the projection is axially aligned with the pressure sensor.

Advantageously, the projection protrudes from a curved region, such that in a closed condition of the locking element, two curved regions are defined configured for not coming into contact with the flexible tube to be placed. In this manner, an area is generated where there is no direct contact with the flexible tube, which enables the original shape thereof to be recovered, i.e., without deformation caused by the projection and the pressure sensor.

Preferably, each of the pressure rollers is freely rotating, comprising a projecting rim at the upper portion and defining a guide region for the flexible tube at the lower portion. Advantageously, the width of the curved region is equal to the height of the guide region, such that when the flexible tube is installed, said guide region covers the entire surface of the tube when it is being pressed and prevents it from moving out of the pressure rollers, which could cause malfunctioning of the fluid supply.

Additionally, anchoring means configured for holding the release means in a closed condition can be provided.

Preferably, these anchoring means may comprise a cover articulated on the casing which has a cavity configured for accommodating the locking element when it is in a closed condition. Therefore, the release means are prevented from unintentionally opening, which would imply that the locking element no longer presses the flexible tube correctly against the rotary pressure rollers and, moreover, prevents vibrations and external movements of the locking element that can cause false readings on the pressure sensor.

The described peristaltic pump therefore represents an innovative structure with structural and constitutional features hitherto unknown for its intended purpose, reasons which, taken together with its usefulness, provide it with sufficient grounds for obtaining the requested exclusivity privilege.

Other features and advantages of the peristaltic pump object of the present invention will be apparent from the description of a preferred, but not exclusive, embodiment, which is illustrated by way of non-limiting example in the accompanying drawings, in which:

### BRIEF DESCRIPTION OF THE FIGURES

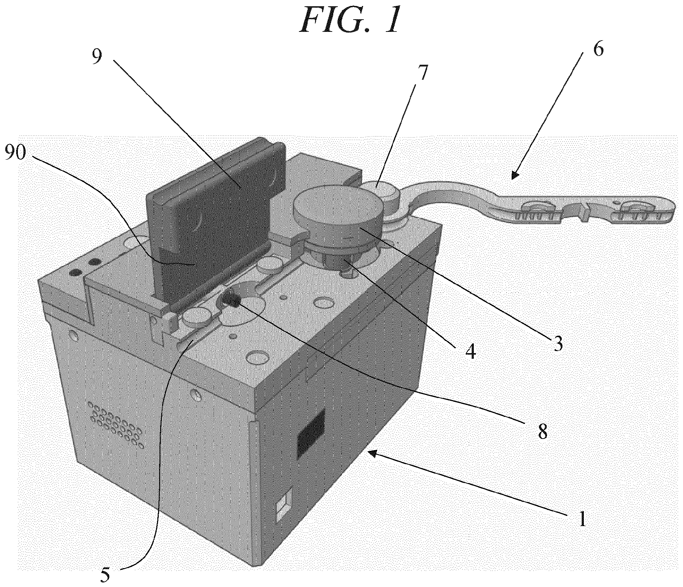

Figure 1 is a perspective view of an embodiment of the peristaltic pump according to the present invention, wherein the release means are in an open condition;
Figure 2 is a perspective view from an upper point of view of the peristaltic pump shown in Figure 1;
Figure 3 is a perspective view of the peristaltic pump wherein the release means are in a closed condition;

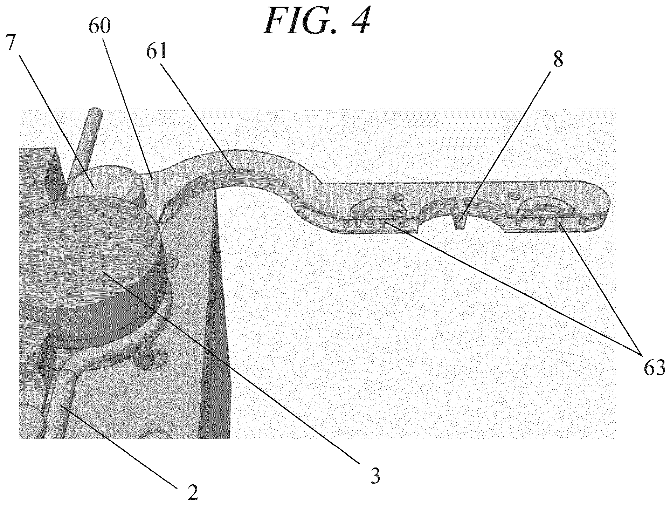

Figure 4 is a partial detailed perspective view of a portion of the peristaltic pump according to the invention;
Figure 5 is a detailed perspective view of the head of the rotor;

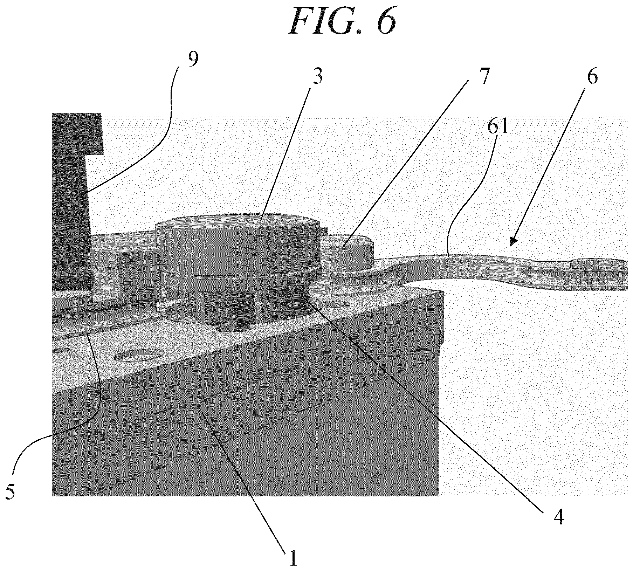

Figure 6 is an additional detailed perspective view of a portion of the peristaltic pump according to the invention;
Figure 7 is a detailed plan view of a portion of the peristaltic pump wherein the pressure detection means are located; and
Figure 8 is a partial plan view of the peristaltic pump wherein the locking element is in a partially closed condition.

### DESCRIPTION OF A PREFERRED EMBODIMENT

In light of the aforementioned figures, and in accordance with the adopted numbering, one may observe therein a preferred exemplary embodiment of the invention, which comprises the parts and elements indicated and described in detail below.

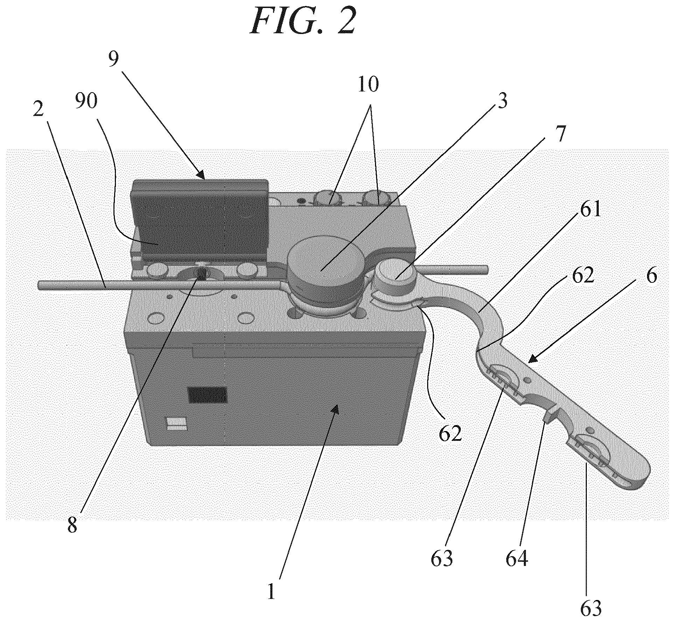

Thus, according to the embodiment represented in the figures, a peristaltic pump comprises a casing (1), made of any suitable material, inside of which conventional drive means are housed, such as an electric motor and a conventional electronic control unit (for which reason it will not be described in further detail in the description thereof) configured for acting on a rotor that will act on a flexible tube (2) (see Figure 2) through which a fluid will circulate.

This rotor is provided at the top with a head (3) that supports a plurality of pressure rollers (4) distributed radially around the rotor, said pressure rollers being intended to make contact with the flexible tube (2) under a certain degree of pressure for the passage of fluid when the pump is running.

Each of the pressure rollers (4) are mounted on the head (3) such that they can rotate freely, comprising a projecting rim (40) at the upper portion and defining a guide region (41) intended for the passage of the flexible tube (2) at the lower portion.

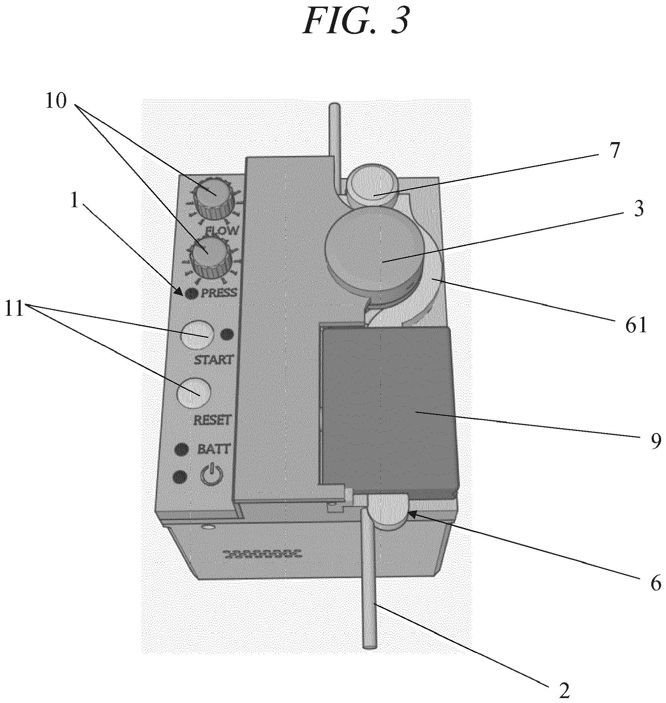

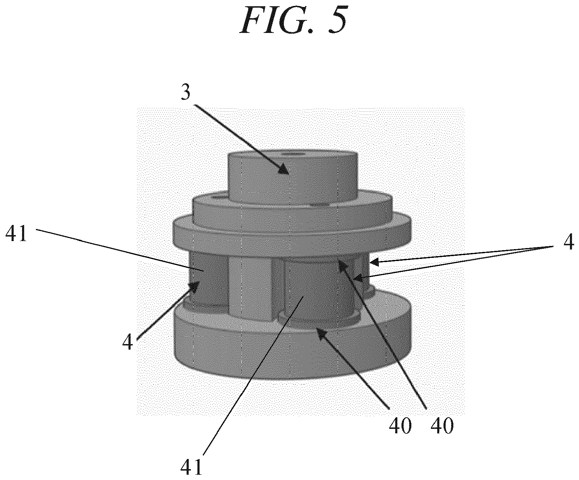

The casing (1) includes a housing, defined by a guide groove (5) with a curved-concave contour running from two opposite ends of the casing, which is configured for accommodating the flexible tube (2). Figure 5 shows a detailed view of the configuration of the head (3). It should be noted that the inlet and outlet section of the guide groove (5) (see Figure 3) are on longitudinal axes parallel to each other, i.e., they follow the same direction, which promotes the use of the peristaltic pump in a vertical arrangement.

Advantageously, to facilitate the placement and removal of the flexible tube (2) in the casing (1), release means for releasing the flexible tube are provided, having an arrangement such that in a closed condition of the release means, such release means are configured to keep the flexible tube (2) in the housing in an immovable manner, as will be detailed later.

Now, going into greater detail, the release means comprise a locking element (6) that is made up of a single part with a generally elongated shape, which is rotatably articulated at a point (7) (also called the articulation axis) of the casing (1) through a coupling section (60) located at one end of the locking element (6). The locking element (6) is made of any suitable material, such as an injection mouldable plastic material, which simplifies the manufacture thereof. The fact that it is plastic material also gives it a certain degree of flexibility in specific areas to favour the correct clamping of the flexible tube (2) without causing damage or unwanted stress during use thereof.

This locking element (6) has a geometric configuration such that it runs along the housing (5) for the flexible tube (2) when it is in a closed position. The coupling section (60) has a substantially semi-annular shape such that it enables the flexible tube (2) to be placed when the locking element (6) is in the open position and, moreover, prevents the flexible tube (2) from coming out of place when the locking element (6) is in the closed position due to external stresses or due to the operation itself.

This locking element (6) has, adjacent to the coupling section (60), a curved region (61) configured for adapting to the contour of the head (3) of the rotor that has a smooth inner surface.

Preferably, the width of this curved region (61) is equal to the height of the guide region (41) of the pressure rollers (4).

The inner portion of the locking element (6) comprises a pair of recessed regions (62), arranged on both sides of the curved region (61), which are configured for partially housing the flexible tube (2). In this manner, a progressive shape change is promoted that enables the pressure roller (4) to neither start nor finish pressing the flexible tube (2) in an abrupt way, thus preventing the formation of folds, vibrations and fatigue on the flexible tube (2).

Therefore, the design of the inner portion of the locking element (6) in the area close to the curved region (61) and the curved region (61) is such that friction zones are created both at the inlet and at the outlet of the rotary head (3) which enables the flexible tube (2) to be tensioned when it rotates in the closing direction to prevent the flexible tube (2) from acquiring folds that could constrict it or from moving linearly while the pressure rollers (4) rotate.

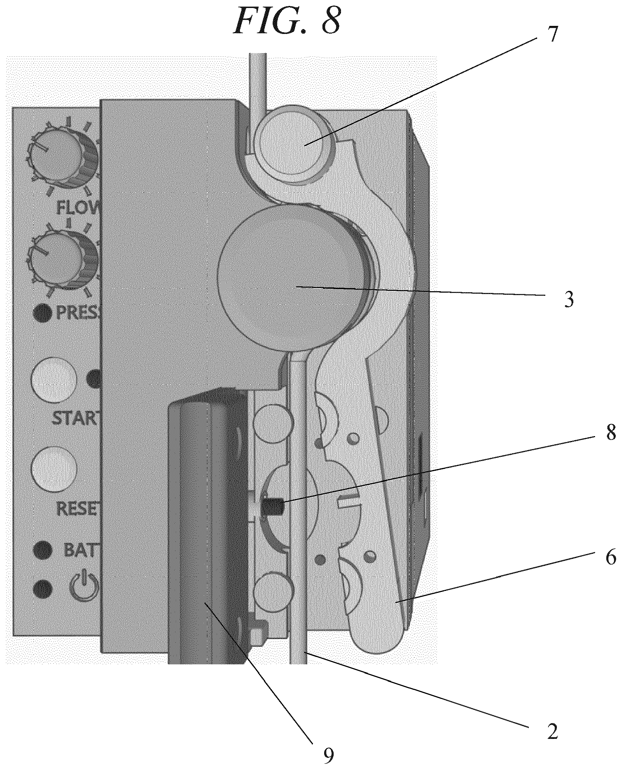

This is achieved by the friction provided by the locking element (6) when closing, on the one hand, at the inlet of the flexible tube (2) with a rotary movement on the rotation axis thereof that pulls the flexible tube (2) outwards and, on the other hand, at the end of the curvature of the curved region (61) which shelters the pressure rollers (4) that exerts friction in the opposite direction to the previous one, as can be seen in Figure 8. Thus, Figure 8 shows that before the locking element (6) is in a completely closed condition or position, the flexible tube (2) is already suitably positioned and tensioned in the area where the pressure rollers (4) are located.

With this closing movement, not only is the flexible tube (2) fixed in the casing (1) of the pump as it passes through the entire stroke, but the flexible tube (2) is tensioned around the head (3) that supports the pressure rollers (4) to prevent folds and constrictions that would cause the pressure rollers (4) to rotate and cause a malfunction.

Furthermore, the locking element (6) includes clamping means configured for clamping the flexible tube (2), which are defined by a pair of partially recessed sections, which are provided on the inner face thereof with a toothed region (63) configured to come into contact with the flexible tube (2) when it is mounted in the guide groove (5) present in the casing (1).

To ensure the correct distribution of the fluid inside the tube, the peristaltic pump includes pressure detection means configured for detecting the pressure value of the fluid to run along the flexible tube (2), the detection means being located in the casing (1).

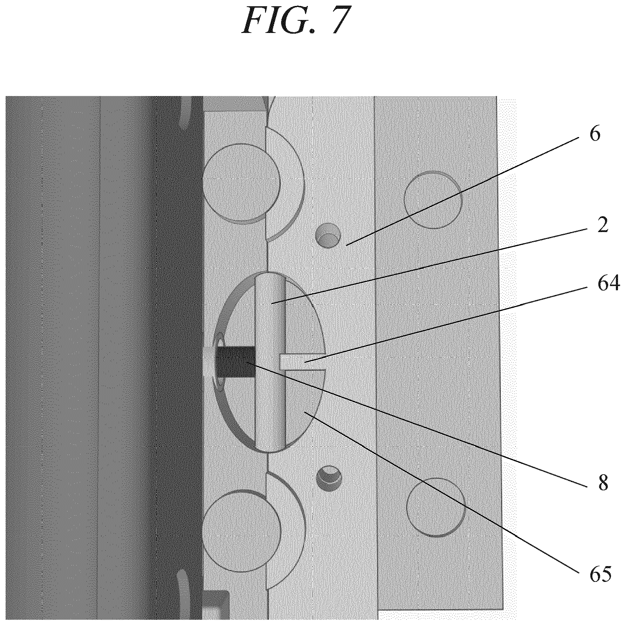

Specifically, and as Figure 7 shows in greater detail, these detection means consist of a pressure sensor (8) (schematically represented) located at a point along the housing intended for the flexible tube (8), the locking element (6) having a projection (64) protruding from the inside thereof, being located such that in a closed condition of the locking element, the projection (64) is axially aligned with the pressure sensor (8).

The pressure sensor (8) calculates the pressure of the internal fluid in the flexible tube by means of the pressure on the outer walls of the flexible tube (2). The reading is made a certain number of times per second and enables, if the pressure at the outlet of the catheter exceeds the pre-defined pressure, the control system to reduce the flow rate until decreasing the pressure automatically. It is worth mentioning that the control system calculates this deformation and tares the pressure system, making it so that the pressure reading is only the one exerted by the fluid inside the flexible tube (2).

It should also be mentioned that the projection (64) protrudes from a curved region formed in the inner portion of the locking element (6), such that in a closed condition of the locking element (6), two curved regions (65) are defined configured for not coming into contact with the flexible tube to be placed, as can be seen in Figure 7, so that in these regions the flexible tube (2) is not subjected to any pressure from the locking element (6).

In order to hold the locking element in a closed condition during the operation of the peristaltic pump, anchoring means are provided that essentially comprise a cover (9) that is hinged at two opposite ends on the casing (1) which has an internal cavity or recess (90), defined by a tiered section, configured for accommodating the locking element (6) when it is in a closed condition.

To manage the control unit of the peristaltic pump drive means, an interface with the user or operator that has a series of rotary pushbuttons (10) and pressure pushbuttons (11) located on the outer portion of the casing (1) is provided. The pump can perform autonomously by means of a battery or be connected to a mains electricity supply.

The details, shapes, dimensions and other accessory elements used to manufacture the peristaltic pump of the invention may be suitably substituted for others which do not depart from the scope defined by the claims included below.

## Claims

1. A peristaltic pump for medical tests, comprising a casing (1) that houses drive means for acting on a rotor provided with a head (3) that supports a plurality of pressure rollers (4) distributed radially around the rotor, said pressure rollers being intended to make contact with pressure with a flexible tube for the passage of a fluid, the casing having a housing configured for accommodating the flexible tube (2), the casing including release means for releasing the flexible tube (2), these release means having an arrangement such that in a closed condition of the release means, such release means are configured to keep the flexible tube (2) in the housing in an immovable manner and the pressure rollers (4) in contact under pressure with the flexible tube (2), the release means comprising a locking element (6) articulated at an articulation point present in the casing (1), having a configuration that runs along the housing for the flexible tube and adapted in shape to the head (3) of the rotor, the locking element (6) including clamping means configured for clamping the flexible tube (2), and including pressure detection means configured for detecting the pressure value of the fluid to run along the flexible tube (2), the detection means being located in the casing (1), **characterised in that** the clamping means comprise at least one section provided on the inner face thereof with a toothed region (63) configured for coming into contact with the flexible tube (2) when it is mounted in the housing present in the casing (1), the detection means consist of a pressure sensor located at a point along the housing for the flexible tube, and the locking element (6) has a projection protruding from the inside thereof, being located such that in a closed condition of the locking element (6), the projection is axially aligned with the pressure sensor, the projection protruding from a curved region, such that in a closed condition of the locking element (6) two curved regions are defined configured for not coming into contact with the flexible tube (2) to be placed.

2. The peristaltic pump according to claim 1, **characterised in that** the locking element has a curved region configured for adapting to the contour of the head (3) of the rotor that has a smooth inner surface.

3. The peristaltic pump according to claim 1, **characterised in that** the inner portion of the locking element (6) comprises a plurality of recessed regions configured for partially housing the flexible tube.

4. The peristaltic pump according to claims 1 and 2, **characterised in that** the clamping means are devoid in the curved region.

5. The peristaltic pump according to claim 1, **characterised in that** anchoring means configured for holding the release means in a closed condition are provided.

6. The peristaltic pump according to claims 1 and 5, **characterised in that** the anchoring means comprise a cover (9) that is articulated on the casing (1), which has a cavity (90) configured for accommodating the locking element (6) when it is in a closed condition.

## Patentansprüche

1. Schlauchpumpe für medizinische Tests, die Folgendes umfasst: ein Gehäuse (1), das Antriebsmittel zum Einwirken auf einen Rotor aufnimmt, der mit einem Kopf (3) versehen ist, der eine Vielzahl von Druckrollen (4) trägt, die radial um den Rotor verteilt sind, wobei die Druckrollen dazu bestimmt sind, mit einem flexiblen Schlauch für den Durchtritt eines Fluids in Druckkontakt zu treten, wobei das Gehäuse eine Aufnahme aufweist, die zur Beherbergung des flexiblen Schlauchs (2) konfiguriert ist, wobei das Gehäuse Freigabemittel zum Freigeben des flexiblen Schlauchs (2) umfasst, wobei diese Freigabemittel eine derartige Anordnung aufweisen, dass derartige Freigabemittel in einem geschlossenen Zustand der Freigabemittel dazu konfiguriert sind, den flexiblen Schlauch (2) in der Aufnahme unbeweglich und die Druckrollen (4) unter Druck mit dem flexiblen Schlauch (2) in Kontakt zu halten, wobei die Freigabemittel ein Verriegelungselement (6) umfassen, das an einem Gelenkpunkt in dem Gehäuse (1) gelenkig angebracht ist, eine Konfiguration aufweist, die entlang der Aufnahme für den flexiblen Schlauch verläuft, und in der Form an den Kopf (3) des Rotors angepasst ist, wobei das Verriegelungselement (6) Einspannmittel enthält, die zum Einspannen des flexiblen Schlauchs (2) konfiguriert sind, und Druckerfassungsmittel enthält, die zum Erfassen des Druckwerts des Fluids, das entlang des flexiblen Schlauchs (2) fließen soll, konfiguriert sind, wobei die Erfassungsmittel in dem Gehäuse (1) gelagert sind, **dadurch gekennzeichnet, dass** die Einspannmittel mindestens einen Abschnitt umfassen, der auf seiner Innenseite mit einem gezahnten Bereich (63) versehen ist, der dazu konfiguriert, mit dem flexiblen Schlauch (2) in Kontakt zu kommen, wenn dieser in der im Gehäuse (1) vorhandenen Aufnahme angebracht ist, die Erfassungsmittel aus einem Drucksensor bestehen, der an einem Punkt entlang der Aufnahme für den flexiblen Schlauch gelagert ist, und das Verriegelungselement (6) einen von seiner Innenseite hervorstehenden Vorsprung aufweist, der so gelagert ist, dass der Vorsprung in einem geschlossenen Zustand des Verriegelungselements (6) axial an dem Drucksensor ausgerichtet ist, wobei der Vorsprung von einem gekrümmten Bereich hervorsteht, sodass in einem geschlossenen Zustand des Verriegelungselements (6) zwei gekrümmte Bereiche definiert sind, die dazu konfiguriert sind, nicht mit dem einzusetzenden flexiblen Schlauch (2) in Kontakt zu kommen.

2. Schlauchpumpe gemäß Anspruch 1, **dadurch gekennzeichnet, dass** das Verriegelungselement einen gekrümmten Bereich aufweist, der zur Anpassung an die Kontur des Kopfes (3) des Rotors, der eine glatte Innenfläche aufweist, konfiguriert ist.

3. Schlauchpumpe gemäß Anspruch 1, **dadurch gekennzeichnet, dass** der innere Abschnitt des Verriegelungselements (6) eine Vielzahl von vertieften Bereichen umfasst, die zur teilweisen Aufnahme des flexiblen Schlauchs konfiguriert sind.

4. Schlauchpumpe gemäß den Ansprüchen 1 und 2, **dadurch gekennzeichnet, dass** in dem gekrümmten Bereich die Einspannmittel fehlen.

5. Schlauchpumpe gemäß Anspruch 1, **dadurch gekennzeichnet, dass** Verankerungsmittel vorgesehen sind, die dazu konfiguriert sind, die Freigabemittel in einem geschlossenen Zustand zu halten.

6. Schlauchpumpe gemäß den Ansprüchen 1 und 5, **dadurch gekennzeichnet, dass** die Verankerungsmittel eine Abdeckung (9) umfassen, die an dem Gehäuse (1) gelenkig angebracht ist und einen Hohlraum (90) aufweist, der zur Aufnahme des Verriegelungselements (6), wenn sich dieses in einem geschlossenen Zustand befindet, konfiguriert ist.

## Revendications

1. Pompe péristaltique pour des tests médicaux, comprenant un boîtier (1) qui loge un moyen d'entraînement pour agir sur un rotor pourvu d'une tête (3) qui supporte une pluralité de rouleaux de pression (4) répartis radialement autour du rotor, lesdits rouleaux de pression étant destinés à entrer en contact avec de la pression avec un tube souple pour le passage d'un fluide, le boîtier ayant un logement conçu pour recevoir le tube souple (2), le boîtier comportant un moyen de libération pour libérer le tube souple (2), ce moyen de libération ayant un agencement tel que, dans un état fermé du moyen de libération, un tel moyen de libération est conçu afin de garder le tube souple (2) dans le logement d'une manière fixe et les rouleaux de pression (4) en contact sous pression avec le tube souple (2), le moyen de libération comprenant un élément de verrouillage (6) articulé au niveau d'un point d'articulation présent dans le boîtier (1), ayant une conception qui s'étend le long du logement pour le tube souple et dont la forme est adaptée à la tête (3) du rotor, l'élément de verrouillage (6) comportant un moyen de serrage conçu pour serrer le tube souple (2) et comportant un moyen de détection de pression conçu pour détecter la valeur de pression du fluide devant s'écouler le long du tube souple (2), le moyen de détection étant situé dans le boîtier (1), **caractérisée en ce que** le moyen de serrage comprend au moins une section pourvue sur sa face interne d'une région dentée (63) conçue pour venir en contact avec le tube souple (2) lorsqu'il est monté dans le logement présent dans le boîtier (1), le moyen de détection est constitué d'un capteur de pression situé au niveau d'un point le long du logement pour le tube souple et l'élément de verrouillage (6) a une partie en saillie dépassant de l'intérieur de celui-ci, qui est située de telle sorte que dans un état fermé de l'élément de verrouillage (6), la partie en saillie est alignée axialement avec le capteur de pression, la partie en saillie dépassant d'une région courbe, de telle sorte que dans un état fermé de l'élément de verrouillage (6), deux régions courbes conçues pour ne pas venir en contact avec le tube souple (2) à placer sont délimitées.

2. Pompe péristaltique selon la revendication 1, **caractérisée en ce que** l'élément de verrouillage a une région courbe conçue pour s'adapter au contour de la tête (3) du rotor qui a une surface interne lisse.

3. Pompe péristaltique selon la revendication 1, **caractérisée en ce que** la partie interne de l'élément de verrouillage (6) comprend une pluralité de régions en creux conçues pour loger partiellement le tube souple.

4. Pompe péristaltique selon les revendications 1 et 2, **caractérisée en ce que** le moyen de serrage est absent dans la région courbe.

5. Pompe péristaltique selon la revendication 1, **caractérisée en ce qu'**un moyen d'ancrage conçu pour maintenir le moyen de libération dans un état fermé est pourvu.

6. Pompe péristaltique selon les revendications 1 et 5, **caractérisée en ce que** le moyen d'ancrage comprend un capot (9) qui est articulé sur le boîtier (1), lequel a une cavité (90) conçue pour recevoir l'élément de verrouillage (6) lorsqu'il est dans un état fermé.
